# EUROPÄISCHE PATENTANMELDUNG

(11) **EP 2 093 284 A1**
(43) Veröffentlichungstag der Anmeldung: **26.08.2009**
(21) Anmeldenummer: 08003054.7
(22) Anmeldetag: 19.02.2008
(51) Int. Cl.: C12N 9/96, C12N 9/06, C12Q 1/32

(54) **Stabilisierung von Dehydrogenasen mit stabilen Coenzymen**

(71) Anmelder: F.HOFFMANN-LA ROCHE AG, 4070 Basel (CH); Roche Diagnostics GmbH, 68305 Mannheim (DE)
(72) Erfinder: Die Erfindernennung liegt noch nicht vor
(74) Vertreter: Weiss, Wolfgang

(57) **Zusammenfassung**

Die vorliegende Erfindung betrifft ein Verfahren zur Stabilisierung eines Enzyms durch Lagerung des Enzyms in Gegenwart eines stabilen Coenzyms. Weiterhin betrifft die vorliegende Erfindung ein mit einem stabilen Coenzym stabilisiertes Enzyms sowie dessen Verwendung in Testelementen zum Nachweis von Analyten.

## Beschreibung

Die vorliegende Erfindung betrifft ein Verfahren zur Stabilisierung eines Enzyms durch Lagerung des Enzyms in Gegenwart eines stabilen Coenzyms. Weiterhin betrifft die vorliegende Erfindung ein mit einem stabilen Coenzym stabilisiertes Enzym sowie dessen Verwendung in Testelementen zum Nachweis von Analyten.

Biochemische Messsysteme sind wichtige Bestandteile klinisch relevanter Analyseverfahren. Hierbei steht die Messung von Analyten, z.B. Metaboliten oder Substraten, im Vordergrund, welche mit Hilfe eines Enzyms direkt oder indirekt bestimmt werden. Die Analyten werden hierbei mit Hilfe eines Enzym-Coenzym-Komplexes umgesetzt und anschließend quantifiziert. Dabei wird der zu bestimmende Analyt mit einem geeigneten Enzym und einem Coenzym in Kontakt gebracht, wobei das Enzym meist in katalytischen Mengen eingesetzt wird. Das Coenzym wird durch die enzymatische Reaktion verändert, z.B. oxidiert bzw. reduziert. Dieser Vorgang kann direkt oder durch einen Mediator elektrochemisch oder photometrisch erfasst werden. Eine Kalibrierung liefert einen direkten Zusammenhang des Messwerts mit der Konzentration des zu bestimmenden Analyten.

Coenzyme sind organische Moleküle, die kovalent oder nicht-kovalent an ein Enzym gebunden sind und durch die Umsetzung des Analyten verändert werden. Prominente Beispiele für Coenzyme sind Nicotinamid-Adenin-Dinukleotid (NAD) bzw. Nicotinamid-Adenin-Dinukleotidphosphat (NADP), aus denen durch Reduktion NADH bzw. NADPH entstehen.

Aus dem Stand der Technik bekannte Messsysteme zeichnen sich durch eine zeitlich begrenzte Haltbarkeit sowie durch spezielle Anforderungen an die Umgebung, wie Kühlung oder trockene Lagerung, zur Erzielung dieser Haltbarkeit aus. Bei bestimmten Anwendungsformen, z.B. bei Tests, die vom Endverbraucher selbst durchgeführt werden, wie etwa beim Blutglucose-Selbstmonitoring, können daher Fehlergebnisse durch falsche, unbemerkte Fehllagerung vorkommen. Besonders die Erschöpfung von Trocknungsmitteln durch zu langes Öffnen der Primärverpackungen kann zu Fehlmessungen führen, die bei manchen Systemen vom Verbraucher kaum zu erkennen sind.

Eine bekannte Maßnahme, die zur Erhöhung der Stabilität von biochemischen Messsystemen eingesetzt wird, ist die Verwendung stabiler Enzyme, z.B. die Verwendung von Enzymen aus thermophilen Organismen. Weiterhin besteht die Möglichkeit, Enzyme durch chemische Modifizierung, z.B. Quervernetzung, oder durch Mutagenese zu stabilisieren. Darüber hinaus können auch Enzymstabilisatoren, wie z.B. Trehalose, Polyvinylpyrrolidon und Serumalbumin, zugesetzt werden oder die Enzyme z.B. durch Photopolymerisation in Polymernetzwerke eingeschlossen werden.

Weiterhin wird versucht, die Haltbarkeit biochemischer Messsysteme durch Verwendung stabiler Mediatoren zu verbessern. So wird durch die Verwendung von Mediatoren mit möglichst niedrigem Redoxpotential die Spezifität von Tests erhöht und Störungen während der Reaktion eliminiert. Eine untere Grenze für das Redoxpotential von Mediatoren bilden jedoch die Redoxpotentiale der Enzym/Coenzymkomplexe. Werden diese unterschritten, wird die Reaktion mit den Mediatoren verlangsamt oder gar unterbunden.

Alternativ können auch biochemische Messsysteme ohne Mediatoren verwendet werden, bei denen beispielsweise eine direkte Detektion von Coenzymen, z.B. des Coenzyms NADH, erfolgt. Ein Nachteil solcher Messsysteme besteht jedoch darin, dass Coenzyme, wie NAD und NADP, instabil sind.

NAD und NADP sind basenlabile Moleküle, deren Abbauwege in der Literatur beschrieben sind (N.J. Oppenheimer in The Pyridine Nucleotide Coenzyms Academic Press New York, London 1982, Hrsg. J. Everese, B. Anderson, K. You, Kapitel 3, Seiten 56-65). Im wesentlichen entsteht beim Abbau von NAD bzw. NADP ADP-Ribose, indem die Glycosyl-Bindungen zwischen der Ribose und der Pyridineinheit gespalten wird. Die reduzierten Formen NADH und NADPH sind hingegen säurelabil: z.B. ist die Epimerisierung ein bekannter Abbauweg. In beiden Fällen liegt der Instabilität von NAD/NADP und NADH/NADPH die Labilität der Glykosyl-Bindung zwischen der Ribose- und der Pyridineinheit zugrunde. Aber auch unter nicht drastischen Bedingungen, wie z.B. in wässriger Lösung, geschieht die Hydrolyse der Coenzyme NAD bzw. NADP allein schon durch die Umgebungsfeuchte. Diese Instabilität kann zu Ungenauigkeiten bei der Messung von Analyten führen.

Eine Reihe von NAD/NADP-Derivaten wird z.B. in B.M. Anderson in the Pyridine Nucleotide Coenzymes, Academic Press New York, London 1982 , Hrsg. J. Everese, B. Anderson, K. You, Kapitel 4 beschrieben. Die meisten dieser Derivate werden allerdings nicht gut von Enzymen akzeptiert. Das einzige Derivat, das daher bisher für diagnostische Tests verwendet wurde, ist 3-Acetylpyridin-Adenin-Dinukleotid (Acetyl NAD), welches erstmals 1956 beschrieben wurde (N.O. Kaplan, J. Biol. Chem. (1956), 221, 823). Auch dieses Coenzym zeigt eine geringe Akzeptanz von Enzymen und eine Änderung im Redoxpotential.

In WO 01/94370 ist die Verwendung weiterer NAD-Derivate mit einer modifizierten Pyridin-Gruppe beschrieben. Modifikationen der Nicotinamid-Gruppe haben jedoch generell direkten Einfluss auf die katalytische Reaktion. In den meisten Fällen ist dieser Einfluss negativ.

In einem weiteren Stabilisierungskonzept wurde die Ribose-Einheit verändert, um dadurch die Stabilität der Glycosyl-Bindung zu beeinflussen. Dieses Vorgehen interferiert nicht direkt mit der katalytischen Reaktion der Nicotinamid-Gruppe. Es kann jedoch ein indirekter Einfluss bestehen, sobald das Enzym eine starke und spezifische Bindung an die Ribose-Einheit aufweist. Kaufmann et al. offenbaren diesbezüglich in WO 98/33936 und US 5,801,006 bzw. in WO 01/49247 eine Reihe von Thioribose-NAD Derivaten. Ein Zusammenhang zwischen der Modifizierung der Nicotinamid-Riboseeinheit und der Aktivität der Derivate in enzymatischen Reaktionen wurde bisher jedoch nicht gezeigt.

carbaNAD, ein Derivat ohne Glycosyl-Bindung, wurde erstmals 1988 beschrieben (J.T. Slama, Biochemistry 1989, 27, 183 und Biochemistry 1989, 28, 7688). Die Ribose wird hierin durch eine carbazyklische Zucker-Einheit substituiert. carbaNAD wurde zwar als Substrat für Dehydrogenasen beschrieben, seine Aktivität wurde bisher jedoch nicht in biochemischen Nachweisverfahren in der Klinik nachgewiesen.

Ein ähnlicher Ansatz wurde später von G.M. Blackburn, Chem. Comm., 1996, 2765 beschrieben, um carbaNAD mit einer Methylenbisphosphonat-Verbindung an Stelle des natürlichen Pyrophosphats herzustellen. Das Methylenbisphosphonat zeigt erhöhte Stabilität gegen Phosphatasen und wurde als Inhibitor für ADP-Ribosylcyclase verwendet. Eine Stabilitätserhöhung gegenüber Hydrolyse war nicht das Ziel (J.T. Slama, G.M. Blackburn).

WO 2007/012494 und US 11/460,366 offenbaren stabile NAD/NADH bzw. NADP/NADPH Derivate, Enzym-Komplexe dieser Derivate und ihre Verwendung in biochemischen Nachweisverfahren und Reagenzienkits.

Die der vorliegenden Erfindung zugrundeliegende Aufgabe war es, Verfahren zur Stabilisierung von Enzymen, insbesondere zur Langzeitstabiliserung von Enzymen bereitzustellen.

Diese Aufgabe wird gelöst durch ein Verfahren zur Stabilisierung eines Enzyms, wobei man das Enzym in Gegenwart eines stabilen Coenzyms lagert. Überraschenderweise wurde gefunden, dass mit Hilfe eines stabilen Coenzyms eine Langzeitstabiliserung von mehreren Wochen bzw. Monaten bei hoher relativer Feuchte oder sogar in flüssiger Phase und bei erhöhten Temperaturen möglich ist. Diese Erkenntnis ist überraschend, da bekannt ist, dass Enzyme in Gegenwart von nativem Coenzym zwar eine erhöhte Kurzzeitstabilität für einige Stunden besitzen (Bertoldi et al., Biochem. J. 389, (2005), 885-898; van den Heuvel et al. (J. Biol. Chem. 280 (2005), 32115-32121; und Pan et al. (J. Chin. Biochem. Soc. Vol. 3 (1974), pp. 1-8), aber eine geringere Haltbarkeit über einen längeren Zeitraum aufweisen (Nutrition Reviews 36 (1978), 251-254).

Gegenüber diesen Erkenntnissen gegenüber dem Stand der Technik war es überraschend, dass ein Enzym in Gegenwart eines stabilen Coenzyms eine deutlich erhöhte Langzeitstabilität als ein Enzym in Gegenwart eines nativen Coenzyms besitzt, insbesondere da die stabilen Coenzyme eine niedrigere Bindungskonstante mit dem Enzym als das native Coenzym besitzen.

Das durch das erfindungsgemäße Verfahren stabilisierte Enzym ist ein Coenyzm-abhängiges Enzym. Geeignete Enzyme sind z.B. Dehydrogenasen, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder Aminosäure-Dehydrogenase, z. B. L-Aminosäure-Dehydrogenase (E.C.1.4.1.5). Weitere geeignete Enzyme sind Oxidasen, wie etwa Glucoseoxidase (E.C.1.1.3.4) oder Cholesterinoxidase (E.C.1.1.3.6) bzw. Aminotransferasen, wie z.B. Aspartat oder Alanin Aminotransferase, 5'-Nukleotidase oder Creatin-Kinase. Vorzugsweise ist das Enzym Glucose-Dehydrogenase.

Das stabile Coenzym ist ein gegenüber dem nativen Coenzym chemisch verändertes Coenzym, welches eine im Vergleich zum nativen Coenyzm höhere Stabilität, (z.B. hydrolytische Stabilität) aufweist. Vorzugsweise ist das stabile Coenyzm unter Testbedingungen gegenüber Hydrolyse stabil. Im Vergleich zum nativen Coenzym kann das stabile Coenzym eine verringerte Bindungskonstante für das Enzym aufweisen, beispielsweise eine um den Faktor von 2 oder mehr verringerte Bindungskonstante.

Bevorzugte Beispiele für stabile Coenzyme sind stabile Derivate von Nicotinamid-Adenin-Dinukleotid (NAD/NADH) oder Nicotinamid-Adenin-Dinukleotidphosphat (NADP/NADPH), oder verkürzte NAD-Derivate, z.B. ohne AMP-Teil bzw. mit nicht nukleosidischen Resten, z.B. hydrophoben Resten.

Bevorzugte stabile Derivate von NAD/NADH bzw. NADP/NADPH sind in den zuvor genannten Referenzen beschrieben, auf deren Offenbarung hiermit ausdrücklich Bezug genommen wird. Besonders bevorzugte stabilisierte Coenzyme sind in WO 2007/012494 bzw. US 11/460,366 beschrieben, auf deren Offenbarung hiermit ausdrücklich Bezug genommen wird. Das stabile Coenzym wird besonders bevorzugt aus Verbindungen mit der allgemeinen Formel (I) oder (11) ausgewählt: 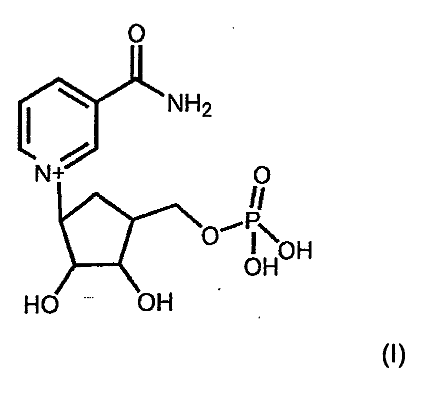 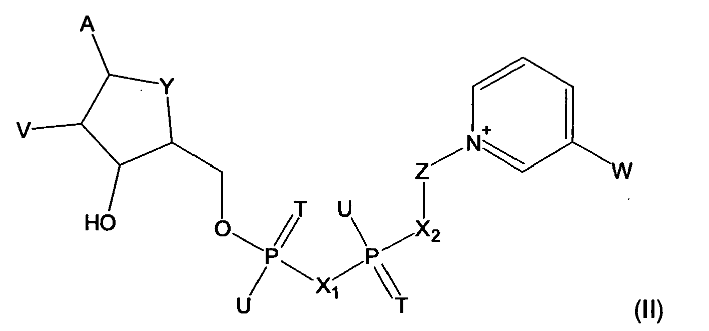 mit
- A =: Adenin oder ein Analog davon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

In den Verbindungen der Formel (I) oder (II) ist Z vorzugsweise ein linearer Rest mit 4-6 C-Atomen, vorzugsweise 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere eine Verbindung der allgemeinen Formel (III) 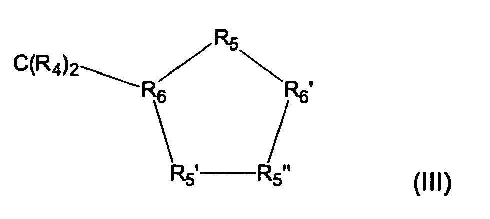
wobei zwischen R⁵' und R⁵" eine Einfach- oder Doppelbindung vorliegen kann, mit
R⁴ = jeweils unabhängig H, F, Cl, CH₃,
R⁵ =CR⁴₂,
wobei R⁵' = O, S, NH, NC₁-C₂-Alkyl, CR⁴₂, CHOH, CHOCH₃, und
R⁵" = CR⁴₂, CHOH, CHOCH₃, wenn zwischen R⁵' und R⁵" eine Einfachbindung vorliegt, und
wobei R⁵'=R⁵"=CR⁴, wenn zwischen R⁵' und R⁵" eine Doppelbindung vorliegt, und
R⁶, R⁶' = jeweils unabhängig CH oder CCH₃.

In einer bevorzugten Ausführungsform enthalten die erfindungsgemäßen Verbindungen Adenin oder Adenin-Analoga, wie z.B. C₈- und N₆-substituiertes Adenin, Deaza-Varianten wie 7-Deaza, Azavarianten wie 8-Aza oder Kombinationen wie 7-Deaza oder 8-Aza oder carbocyclische Analoga, wie Formycin, wobei die 7-Deazavarianten in der 7-Position mit Halogen, C₁-C₆-Alkinyl, -Alkenyl oder -Alkyl substituiert sein können.

In einer weiteren bevorzugten Ausführungsform enthalten die Verbindungen Adenosin-Analoga, welche statt Ribose z.B. 2-Methoxy-deoxyribose, 2'-Fluoro-deoxyribose, Hexitol, Altritol bzw. polycyclische Analoga, wie Bicyclo-, LNA- und Tricyclo-Zucker enthalten.

Insbesondere können in den Verbindungen der Formel (I) oder (II), auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S.

In den erfindungsgemäßen Verbindungen der Formel (I) oder (II) ist W vorzugsweise CONH₂ oder COCH₃.

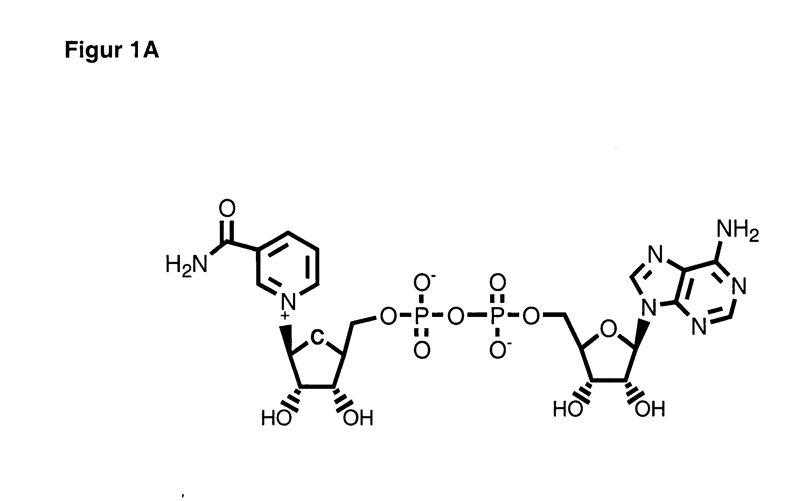

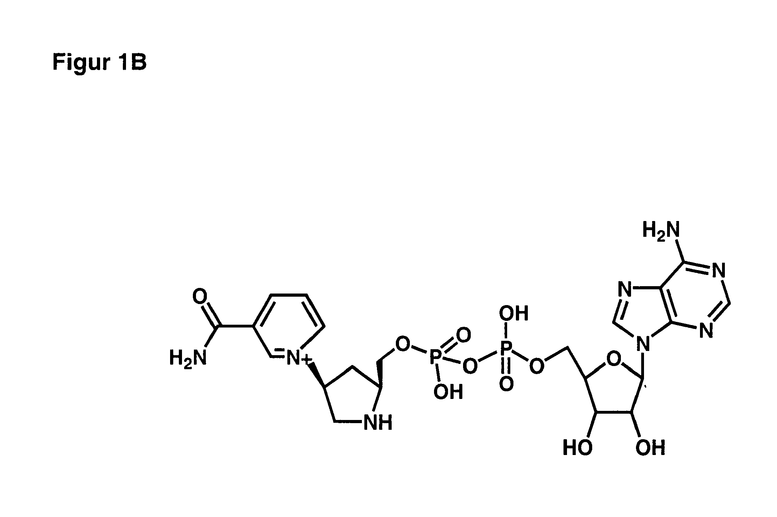

In den Gruppen der Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂. Spezifische Beispiele für bevorzugte stabilisierte Coenzyme sind in Figur 1A und B abgebildet.

Das erfindungsgemäße Verfahren ist insbesondere zur Langzeitstabilisierung von Enzymen geeignet. Dies bedeutet, dass man das mit einem stabilen Coenzym stabilisierte Enzym, z.B. als Trockensubstanz, beispielsweise über eine Dauer von mindestens 2 Wochen, vorzugsweise von mindestens 4 Wochen und besonders bevorzugt von mindestens 8 Wochen lagert und wobei die Enzymaktivität vorzugsweise um weniger als 50 %, besonders bevorzugt weniger als 30 % und am meisten bevorzugt um weniger als 20 % bezüglich des Ausgangswerts der Enzymaktivität abnimmt.

Weiterhin umfasst das erfindungsgemäße Verfahren eine Lagerung des mit einem stabilen Coenzym stabilisierten Enzyms bei erhöhten Temperaturen, beispielsweise bei einer Temperatur von mindestens 20 °C, vorzugsweise von mindestens 25 °C und besonders bevorzugt von mindestens 30 °C. Die Enzymaktivität nimmt dabei vorzugsweise um weniger als 50 %, besonders bevorzugt weniger als 30 % und am meisten bevorzugt weniger als 20 % bezüglich ihres Ausgangswerts ab.

Durch die erfindungsgemäße Stabilisierung ist es möglich, das mit einem stabilen Coenzym stabilisierte Enzym auch ohne Trocknungsreagenz für eine lange Zeit, wie oben angegeben, und/oder bei hohen Temperaturen, wie oben angegeben, zu lagern. Weiterhin kann das stabilisierte Enzym auch bei einer hohen relativen Luftfeuchtigkeit, z.B. einer relativen Luftfeuchtigkeit von mindestens 50 % gelagert werden, wobei die Enzymaktivität vorzugsweise um weniger als 50 %, besonders bevorzugt weniger als 30 % und am meisten bevorzugt weniger als 20 % bezüglich des Ausgangswerts abnimmt.

Die Lagerung des mit einem stabilen Coenzym stabilisierten Enzyms kann einerseits als Trockensubstanz und andererseits in Flüssigphase erfolgen. Bevorzugt erfolgt die Lagerung des stabilisierten Enzyms auf oder in einem Testelement, das zur Bestimmung eines Analyten geeignet ist. Das mit einem stabilen Coenzym stabilisierte Enzym ist dabei vorzugsweise Bestandteil eines Nachweisreagenz, welches gegebenenfalls noch weitere Bestandteile wie etwa Salze, Puffer, etc. enthalten kann. Vorzugsweise ist das Nachweisreagenz dabei frei von einem Mediator.

Das mit einem stabilen Coenzym stabilisierte Enzym kann zum Nachweis von Analyten, beispielsweise Parametern in Körperflüssigkeiten wie etwa Blut, Serum, Plasma oder Urin bzw. in Abwasserproben oder Lebensmitteln eingesetzt werden.

Als Analyten können beliebige biologische oder chemische Substanzen bestimmt werden, die durch eine Redoxreaktion nachgewiesen werden können, z.B. Substanzen, bei denen es sich um Substrate eines Coenzymabhängigen Enzyms handelt oder Coenzym-abhängige Enzyme selbst. Bevorzugte Beispiele für Analyten sind Glucose, Milchsäure, Äpfelsäure, Glycerin, Alkohol, Cholesterin, Triglyceride, Ascorbinsäure, Cystein, Glutathion, Peptide, Harnstoff, Ammonium, Salicylat, Pyruvat, 5'-Nukleotidase, Creatinkinase (CK), Lactat-Dehydrogenase (LDH), Kohlendioxid etc. Bevorzugt ist der Analyt Glucose.

Ein weiterer Gegenstand der vorliegenden Erfindung ist die Verwendung einer erfindungsgemäßen Verbindung oder eines erfindungsgemäßen, mit einem stabilen Coenzym stabilisierten Enzyms zum Nachweis eines Analyten in einer Probe durch eine enzymatische Reaktion. Besonders bevorzugt ist hierbei der Nachweis von Glucose mit Hilfe von Glucose-Dehydrogenase (GlucDH).

Die Veränderung des stabilen Coenzyms durch Reaktion mit dem Analyten kann grundsätzlich auf beliebige Art und Weise nachgewiesen werden. Hier können grundsätzlich alle aus dem Stand der Technik bekannten Methoden zum Nachweis enzymatischer Reaktionen eingesetzt werden. Vorzugsweise wird jedoch die Veränderung des Coenzyms durch optische Methoden nachgewiesen. Optische Nachweismethoden umfassen beispielsweise die Messung von Absorption, Fluoreszenz, Circulardichroismus (CD), optische Rotationsdispersion (ORD), Refraktometrie etc. Besonders bevorzugt wird die Veränderung des Coenzyms durch Messung der Fluoreszenz nachgewiesen. Die Fluoreszenzmessung ist hochsensitiv und ermöglicht den Nachweis selbst geringer Konzentrationen des Analyten in miniaturisierten Systemen.

Zum Nachweis eines Analyten kann ein Flüssigtest verwendet werden, wobei das Reagenz z.B. in Form einer Lösung oder Suspension in einer wässrigen oder nichtwässrigen Flüssigkeit oder als Pulver oder Lyophilisat vorliegt. Es kann jedoch auch ein Trockentest verwendet werden, wobei das Reagenz auf einem Träger, einem Teststreifen, aufgebracht ist. Der Träger kann beispielsweise einen Teststreifen, umfassend ein saugfähiges oder/und quellbares Material, umfassen, das von der zu untersuchenden Probeflüssigkeit benetzt wird.

Ein besonders bevorzugtes Testformat, umfasst die Verwendung des Enzyms Glucose-Dehydrogenase mit einem stabilen NAD-Derivat zum Nachweis von Glucose, wobei ein Derivat des reduzierten Coenzyms NADH gebildet wird. Der Nachweis von NADH erfolgt durch optische Methoden, z.B. durch photometrische oder fluorometrische Bestimmung nach UV-Anregung. Ein besonders bevorzugtes Testsystem ist in US 2005/0214891 beschrieben, auf das hier ausdrücklich Bezug genommen wird.

Noch ein weiterer Gegenstand der vorliegenden Erfindung ist ein mit einem stabilen Coenzym stabilisiertes Enzym, wobei das stabilisierte Enzym bei einer Lagerung von vorzugsweise mindestens 2 Wochen, besonders bevorzugt mindestens 4 Wochen und am meisten bevorzugt mindestens 8 Wochen bei einer Temperatur von vorzugsweise mindestens 20 °C, besonders bevorzugt mindestens 25 °C und am meisten bevorzugt mindestens 30 °C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

Noch ein weiterer Gegenstand der Erfindung ist ein Nachweisreagenz zur Bestimmung eines Analyten, welches ein mit einem stabilen Coenzym stabilisiertes Enzym, wie zuvor angegeben, enthält. Außerdem betrifft die Erfindung ein Testelement, welches ein erfindungsgemäßes stabilisiertes Enzym bzw. ein erfindungsgemäßes Nachweisreagenz enthält. Das Nachweisreagenz bzw. das Testelement können durch Durchführung von Trocken- oder Flüssigtests geeignet sein. Bevorzugt ist das Testelement ein Teststreifen zum fluorometrischen oder photometrischen Nachweis eines Analyten. Ein solcher Teststreifen enthält das mit einem stabilen Coenzym stabilisierte Enzym immobilisiert auf einem saugfähigen oder/und quellbaren Material, wie etwa Cellulose, Kunstoffe, etc.

Die Erfindung soll durch die nachfolgenden Figuren und Beispiele näher erläutert werden:

### Beschreibung der Figuren

**Figur 1A****:** Darstellung des stabilen Coenzym carba-NAD (cNAD).
**Figur 1B****:** Darstellung des stabilen Coenzyms Pyrrolidinyl-NAD.
**Figur 2****:** Darstellung der Ergebnisse der Enzymkinetik von Glucose-Dehydrogenase in Gegenwart von NAD bzw. von Glucose-Dehydrogenase in Gegenwart von cNAD vor und nach Lagerung.
   **2A:** Kinetik von GlucDH in Gegenwart von NAD nach 1 Tag.
   **2B:** Kinetik von GlucDH in Gegenwart von cNAD nach 1 Tag.
   **2C:** Kinetik von GlucDH in Gegenwart von NAD nach 5 Wochen Lagerung bei 32 °C und 85 % relativer Luftfeuchtigkeit.
   **2D:** Kinetik von GlucDH in Gegenwart von cNAD nach 5 Wochen Lagerung bei 32 °C und 85 % relativer Luftfeuchtigkeit.

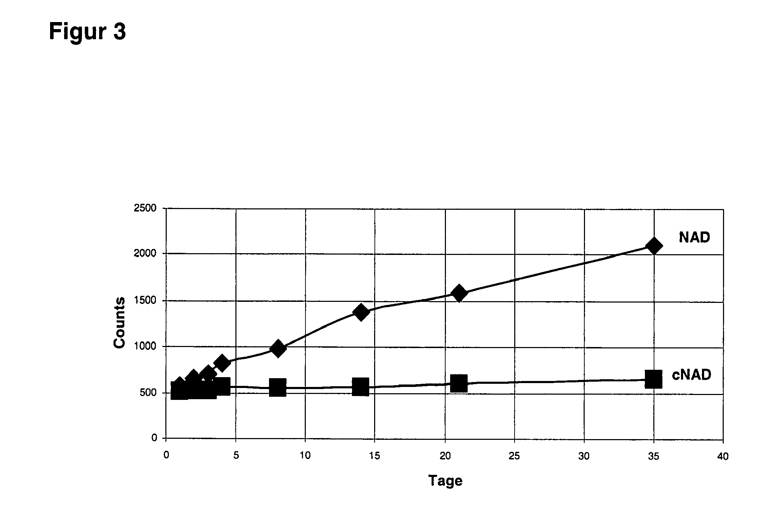

**Figur 3****:** Vergleich der Blankwerte von Glucose-Dehydrogenase in Gegenwart von NAD bzw. von GlucDH in Gegenwart von cNAD über einen Zeitraum von bis zu 5 Wochen bei 32 °C und 85 % Luftfeuchtigkeit.

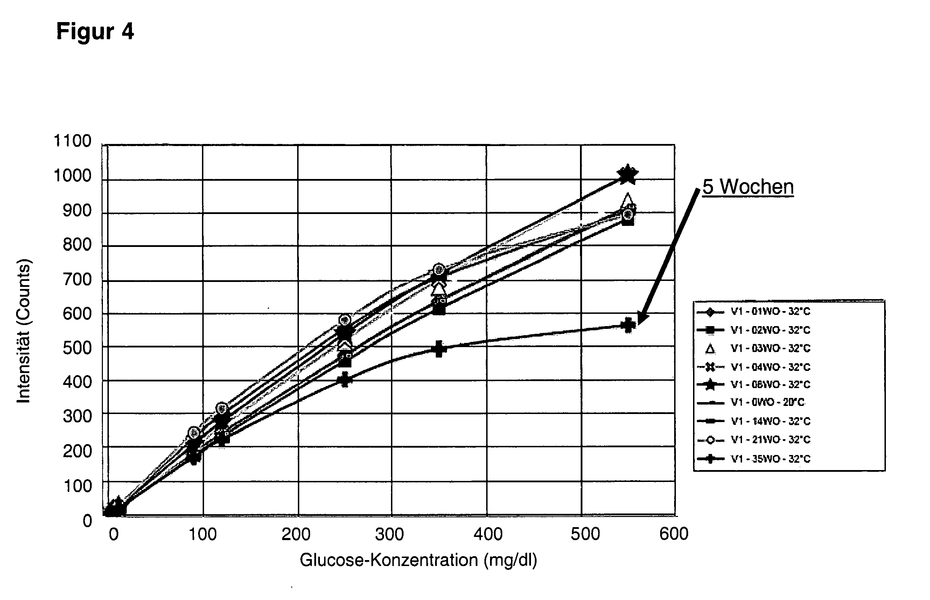

**Figur 4****:** Darstellung verschiedener Funktionskurven von Glucose-Dehydrogenase nach Lagerung von Glucose-Dehydrogenase in Gegenwart von NAD bei 32 °C und 85 % Luftfeuchtigkeit. Die Lagerungsdauer variierte zwischen 1 Tag und 5 Wochen.

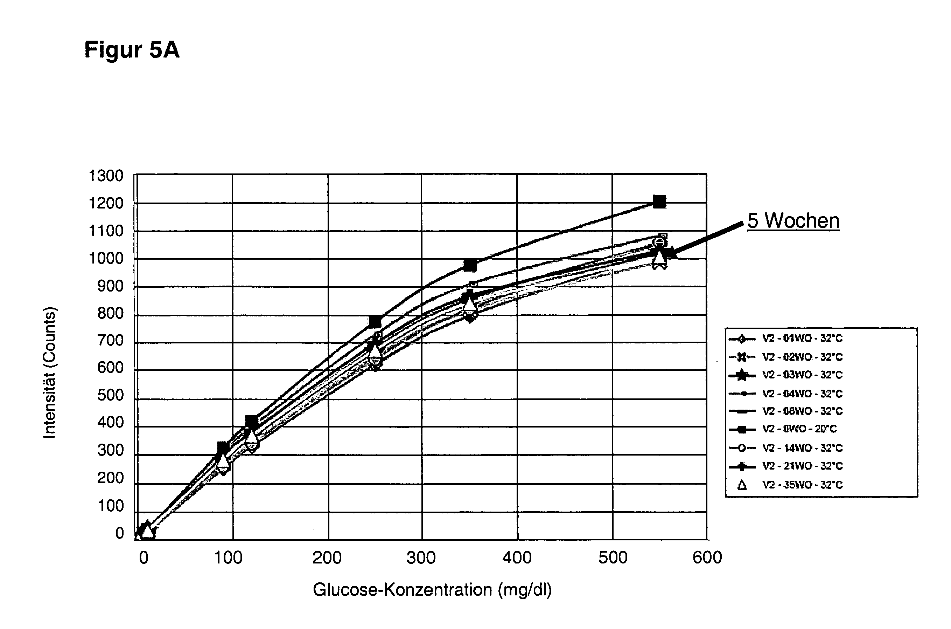

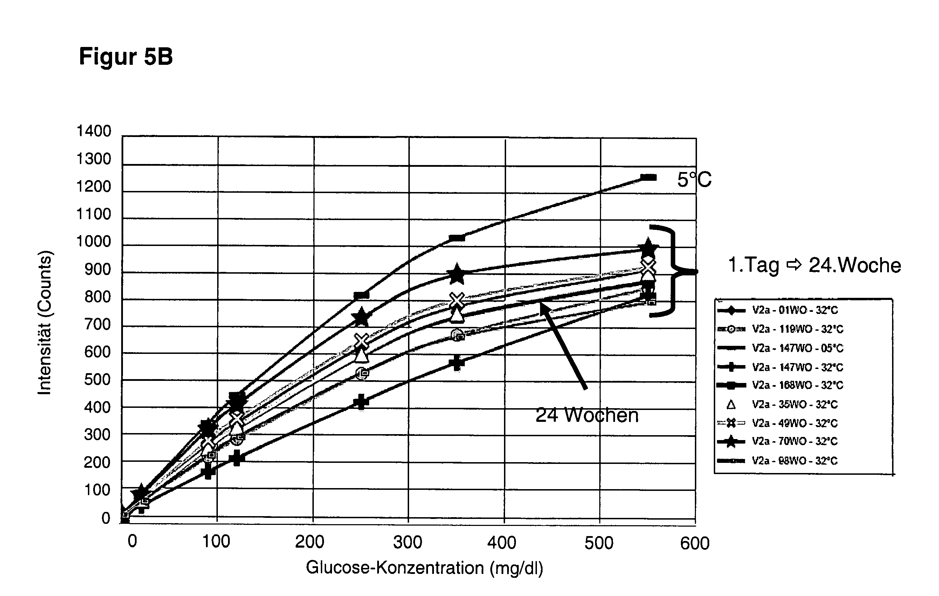

**Figur 5****:** Darstellung verschiedener Funktionskurven von Glucose-Dehydrogenase nach Lagerung von Glucose-Dehydrogenase in Gegenwart von cNAD bei 32 °C und 85 % Luftfeuchtigkeit. Die Lagerungsdauer variierte zwischen 1 Tag und 5 Wochen (Figur 5A) bzw. zwischen 1 Tag und 24 Wochen (Figur 5B).

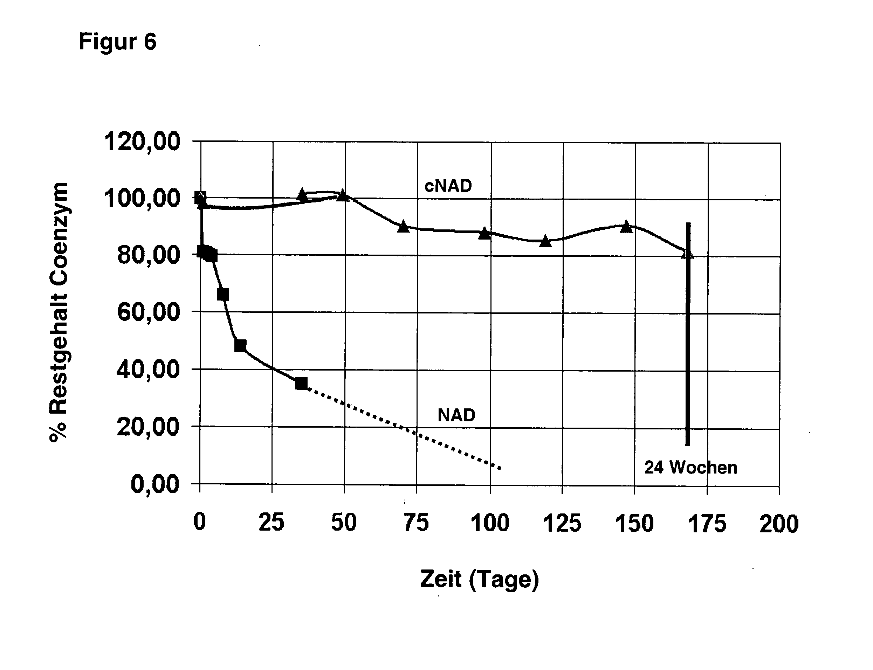

**Figur 6****:** Darstellung des Restgehaltes an NAD bzw. cNAD nach Lagerung von Glucose-Dehydrogenase in Gegenwart von NAD bzw. cNAD über 24 Wochen bei 32 °C und 85 % Luftfeuchtigkeit.

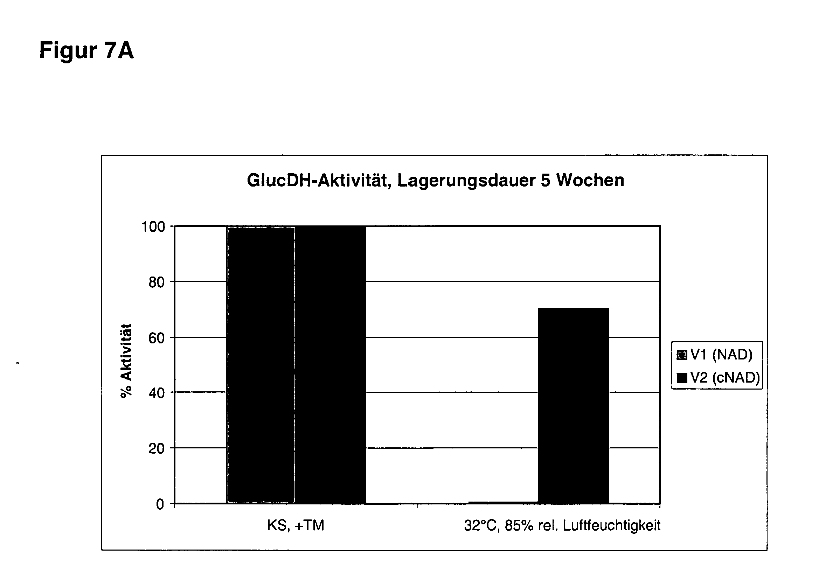

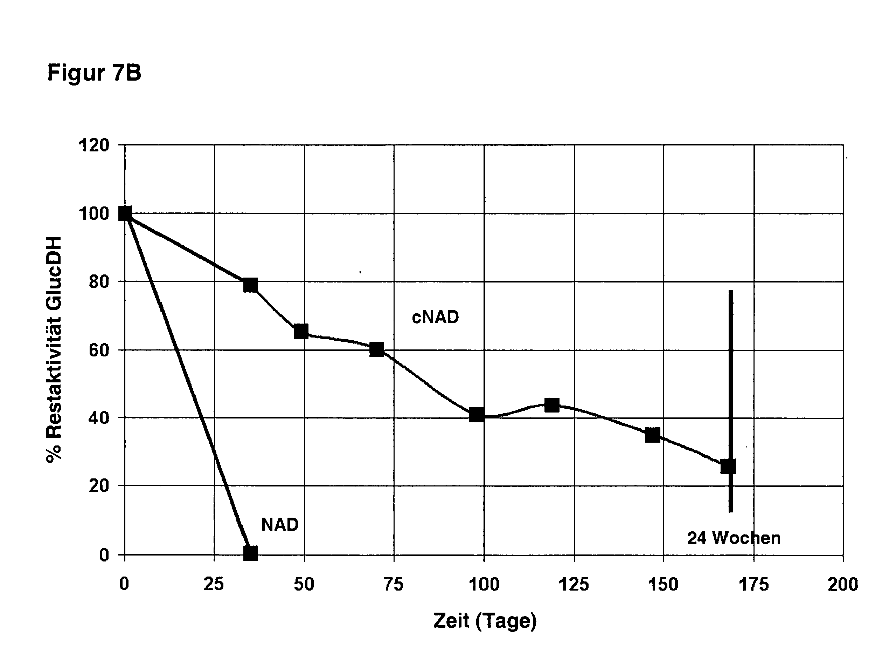

**Figur 7****:** Darstellung der GlucDH-Aktivität nach Lagerung von Glucose-Dehydrogenase in Gegenwart von NAD bzw. cNAD über 5 Wochen (Figur 7A) bzw. 24 Wochen (Figur 7B) bei 32 °C und 85 % Luftfeuchtigkeit.

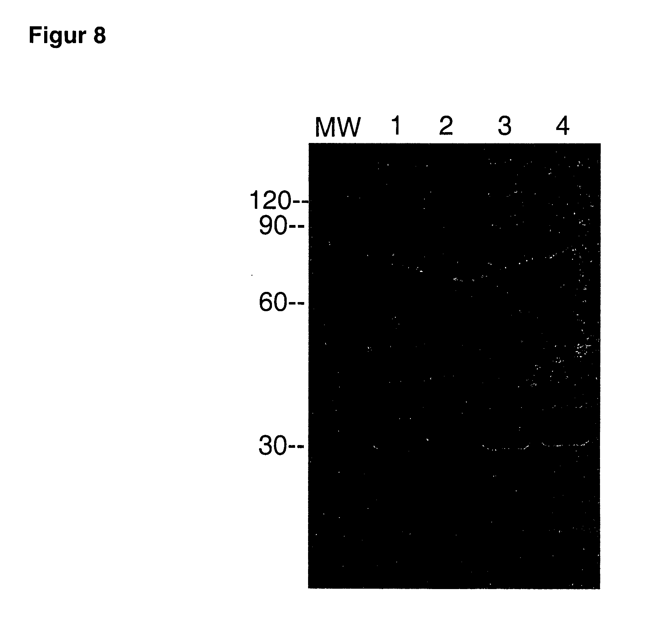

**Figur 8****:** Gelelektrophoretische Analyse von Glucose-Dehydrogenase nach Lagerung in Gegenwart von NAD bzw. cNAD. Testbedingungen: MW, 10-220 kDa-Marker; 1: GlucDH/NAD, 5 Wochen bei 6 °C; 2: GlucDH/NAD, 5 Wochen bei 32 °C/85 % relative Luftfeuchtigkeit; 3: GlucDH/cNAD, 5 Wochen bei 6 °C; 4: GlucDH/cNAD, 5 Wochen bei 32 °C/85 % relative Luftfeuchtigkeit.

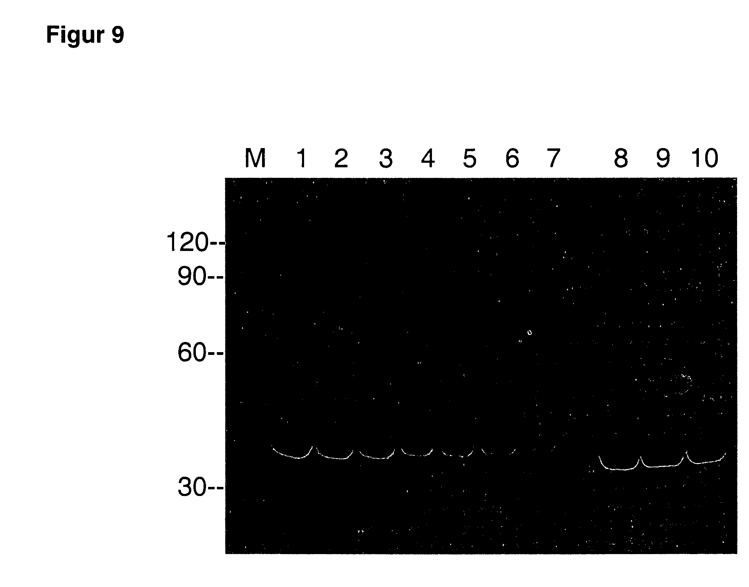

**Figur 9****:** Gelelektrophoretische Analyse von Glucose-Dehydrogenase nach Lagerung bei 50 °C in Gegenwart von NAD bzw. cNAD. Testbedingungen: MW, 10-220 kDa-Marker; 1: GlucDH 8,5 mg/ml, NAD, 0 Stunden; 2: GlucDH 8,5 mg/ml, NAD, 22 Stunden; 3: GlucDH 8,5 mg/ml, NAD, 96 Stunden; 4: GlucDH 8,5 mg/ml, NAD, 118 Stunden; 5: GlucDH 8,5 mg/ml, NAD, 140 Stunden; 6: GlucDH 8,5 mg/ml, NAD, 188 Stunden; 7: GlucDH 8,5 mg/ml, NAD, 476 Stunden; 8: GlucDH 8,5 mg/ml, cNAD, 0 Stunden; 9: GlucDH 8,5 mg/ml, cNAD, 188 Stunden; 10: GlucDH 8,5 mg/ml, cNAD, 476 Stunden.

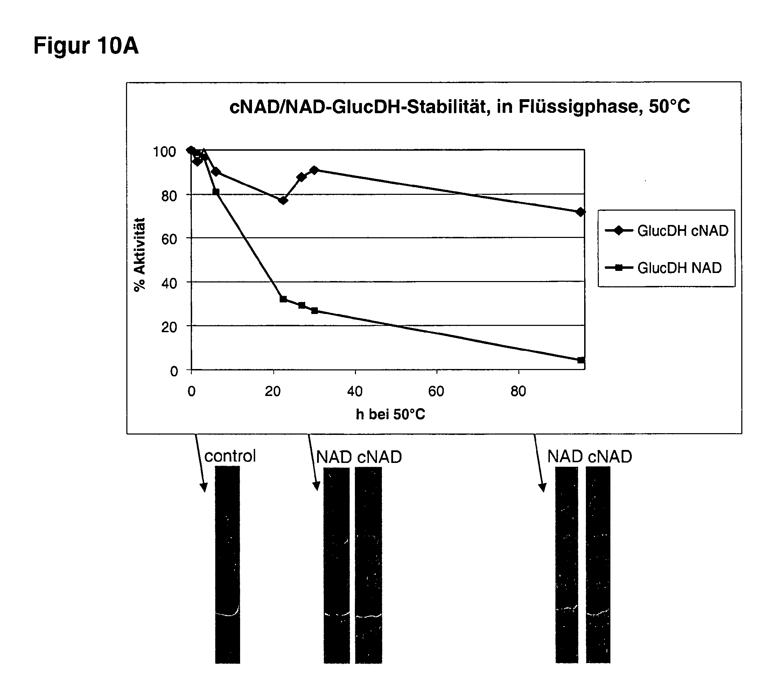

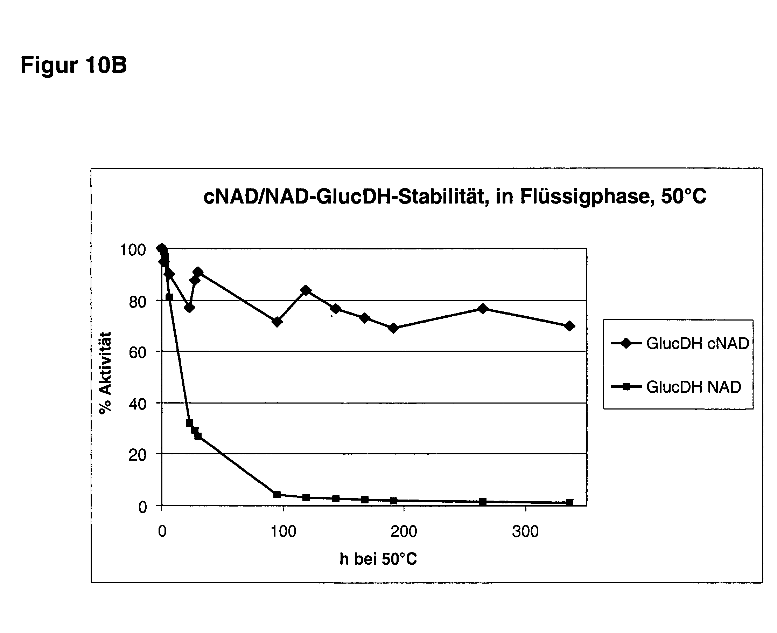

**Figur 10****:** Darstellung der Stabilität von Glucose-Dehydrogenase in Gegenwart von NAD bzw. cNAD in Flüssigphase bei 50 °C über einen Zeitraum von 4 Tagen (Figur 10A) bzw. 14 Tagen (Figur 10B). Testbedingungen: GlucDH 10 mg/ml; NAD bzw. cNAD 12 mg/ml; Puffer: 0,1 M Tris, 1,2 M NaCl, pH 8,5; Temperatur 50 °C.

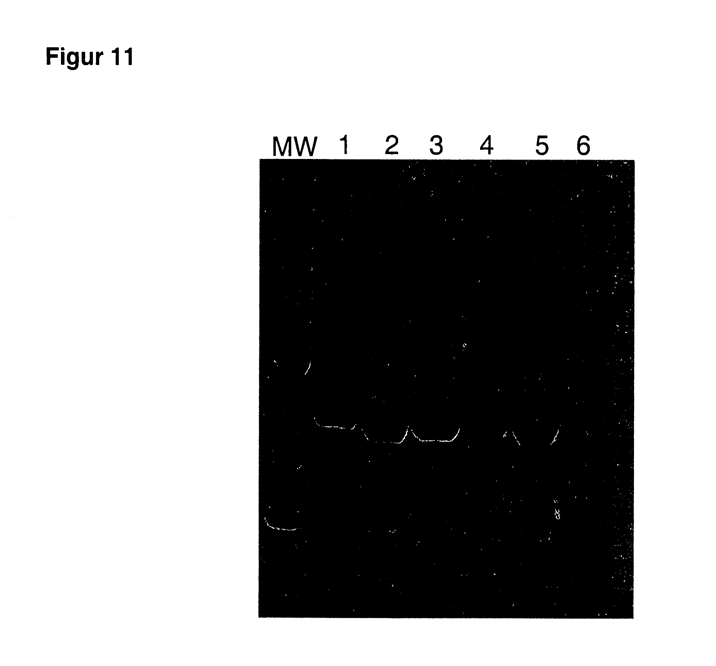

**Figur 11****:** Gelelektrophoretische Analyse von Alkohol-Dehydrogenase aus Hefe nach Lagerung in Gegenwart von NAD bzw. cNAD. Testbedingungen: MW, 10-220 kDa-Marker; 1: ADH, 65 Stunden bei 6 °C; 2: ADH/cNAD, 65 Stunden bei 6 °C; 3: ADH/NAD, 65 Stunden bei 6 °C; 4: ADH, 65 Stunden bei 35 °C; 5: ADH/cNAD, 65 Stunden bei 35 °C; 6: ADH/NAD, 65 Stunden bei 35 °C.

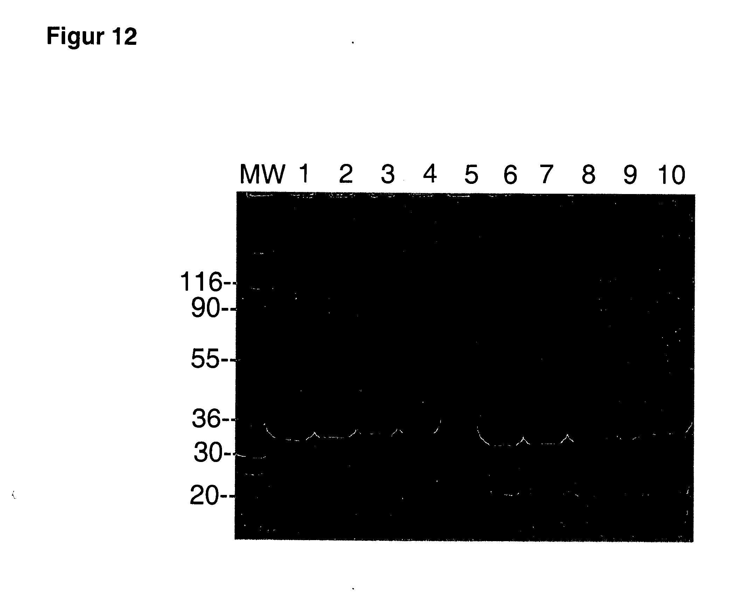

**Figur 12****:** Gelelektrophoretische Analyse von Alkohol-Dehyrogenase aus Hefe nach Lagerung bei 35 °C in Gegenwart von NAD bzw. cNAD. Testbedingungen: MW, 10-220 kDa-Marker; 1: ADH/NAD, 0 Tage; 2: ADH/NAD, 1 Tag; 3: ADH/NAD, 2 Tage; 4: ADH/NAD, 3 Tage; 5: ADH/NAD,
5 Tage; 6: ADH/cNAD, 0 Tage; 7: ADH/cNAD, 1 Tag; 8: ADH/cNAD, 2 Tage; 9: ADH/cNAD, 3 Tage; 10: ADH/cNAD, 6 Tage.

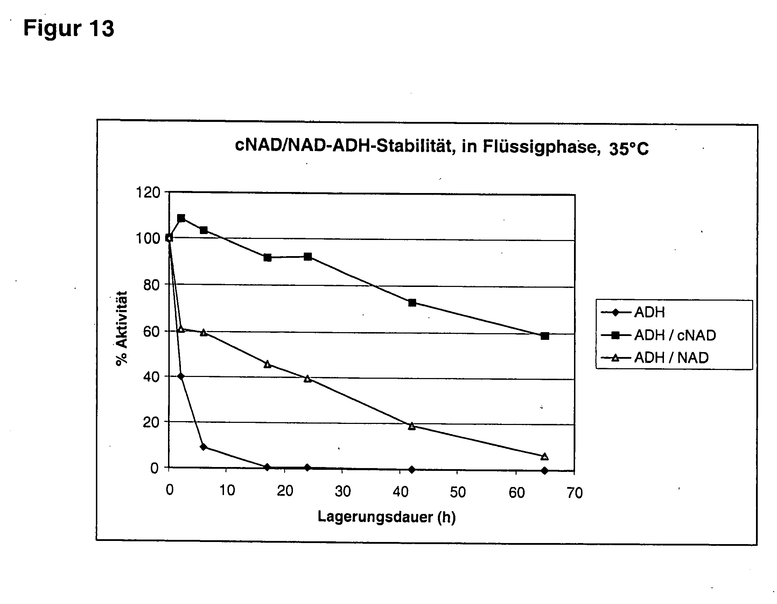

**Figur 13****:** Darstellung der Stabilität von Alkohol-Dehydrogenase aus Hefe in Gegenwart von NAD bzw. cNAD in Flüssigphase bei 35 °C über einen Zeitraum von 65 Stunden. Testbedingungen: ADH 5 mg/mi; NAD bzw. cNAD 50 mg/ml; Puffer: 75 mM Na₄P₂O₇, Glycin, pH 9,0; Temperatur 35 °C.

### Beispiel 1

Der Glucose-spezifischen GlucDH wurden carba-NAD (Fig. 1A) oder NAD zugesetzt. Diese Rezepturen wurden jeweils auf Pokalonfolie (Lonza) aufgetragen und nach Trocknung unter warmen und feuchten Bedingungen (32 °C, 85 % relative Luftfeuchtigkeit) eingelagert. Anschließend wurden in regelmäßigen Abständen die Reaktionskinetik sowie die Funktionskurve bestimmt. Parallel wurde zu den jeweiligen Messterminen eine cNAD/NAD-Analytik sowie eine Bestimmung der Restaktivität des Enzyms vorgenommen.

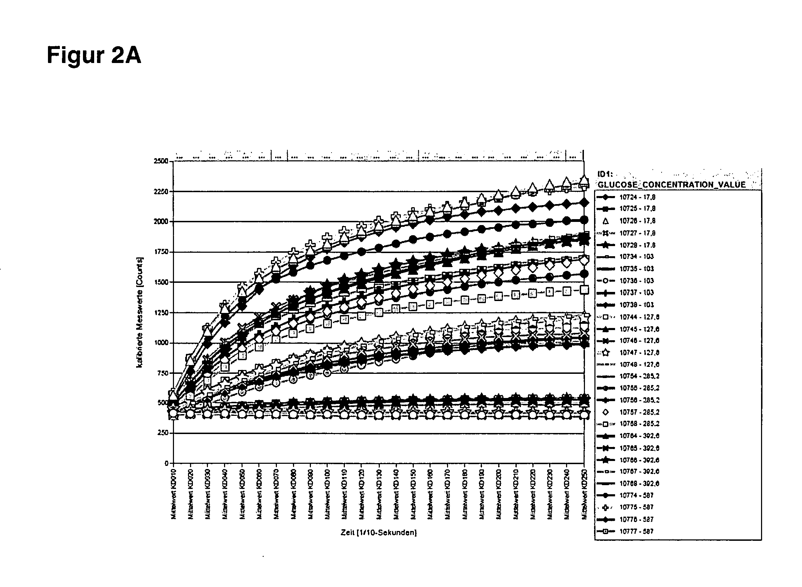

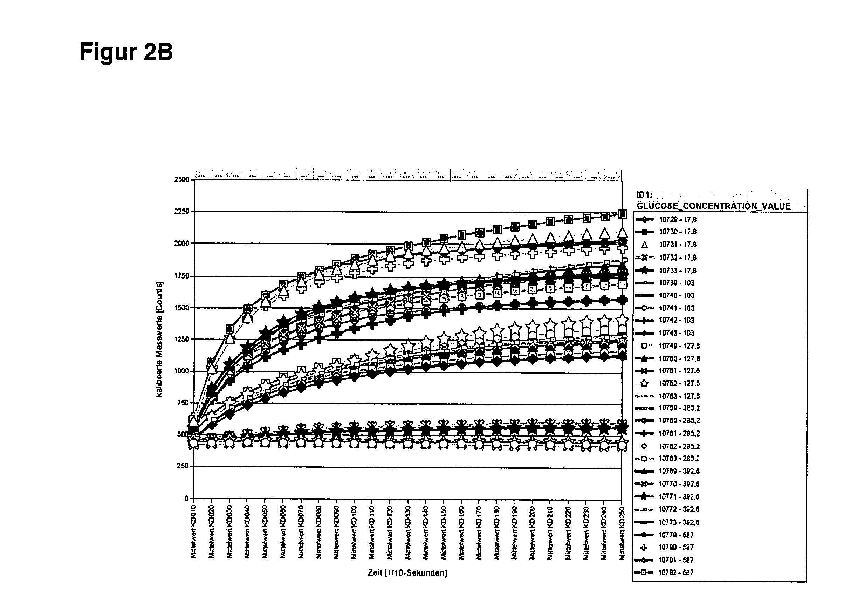

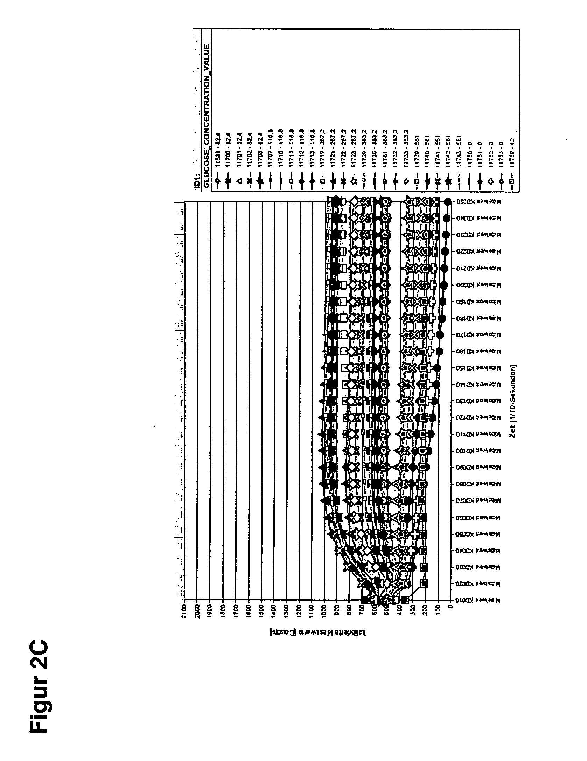

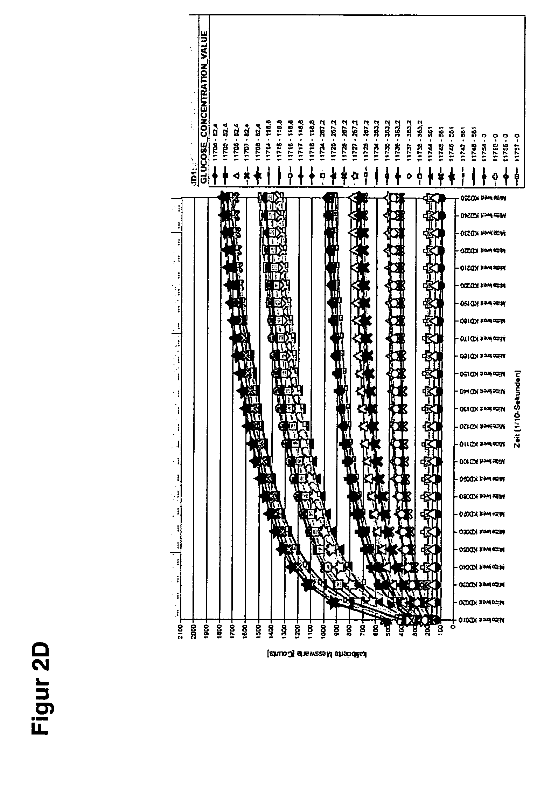

Die am ersten Tag bestimmten Kinetik-Kurven für NAD (Figur 2A) und cNAD (Figur 2B) sind vergleichbar und zeigen auch einen ähnlichen Hub in der Glucoseabhängigkeit. Nach 5 Wochen ist jedoch ein deutlicher Unterschied in den Kinetik-Kurven zu erkennen. Während die Kinetik für NAD (Figur 2C) stark in ihrer Dynamik nachlässt, bleibt die Kinetik des mit cNAD stabilisierten Enzyms praktisch unverändert (Figur 2D).

Auch in den Blankwerten (Trockenleerwert vor Auftrag einer Blutprobe) zeigt sich ein deutlicher Unterschied, wie aus Figur 3 ersichtlich ist. Der Anstieg des Trockenleerwerts bei NAD ist auf die Bildung von fluoreszierenden Teilchen zurückzuführen (Oppenheimer (1982), Supra). Überraschenderweise geschieht dies nicht bei cNAD.

Die unterschiedliche Stabilität von Glucose-Dehydrogenase in Gegenwart von NAD bzw. cNAD ist auch aus einem Vergleich der Figuren 4 und 5 ersichtlich. Nach 5 Wochen liegt die Funktionskurve bei dem mit cNAD stabilisierten Enzym noch in der Schar der voherigen Messungen (Figur 5A), während bei dem mit NAD versetzten Enzym (Figur 4) die Kurve bei höheren Konzentrationen abknickt, was ein typisches Zeichen für zu geringe Mengen an Enzym/Coenzym ist. Figur 5B zeigt verschiedene Funktionskurven der mit cNAD stabilisierten Glucose-Dehydrogenase über einen Zeitraum von 24 Wochen. In diesem Zusammenhang wird deutlich, dass sich die Funktion des Enzyms bei hohen Glucose-Konzentrationen über den gesamten Zeitraum hinweg nur geringfügig verändert und nach 24 Wochen etwa dem nach 5 Wochen erhaltenen Wert entspricht.

Die Beziehung zwischen Struktur des Coenzyms und dessen Stabilität über einen vorbestimmten Zeitraum ergibt sich aus Figur 6. Demnach beträgt der Restgehalt an cNAD in einem Glucose-Nachweisreagenz nach 24 Wochen Lagerung (bei 32 °C und 85 % relativer Luftfeuchtigkeit) noch etwa 80 % des Ausgangswertes, während sich der Gehalt an NAD in einem mit NAD stabilisierten Glucose-Nachweisreagenz bereits nach 5 Wochen auf etwa 35 % des Ausgangswertes verringert und gemäß Extrapolation nach etwa 17 Wochen auf null reduziert.

Vollkommen überraschend ist das Ergebnis der Restaktivitätsbestimmung des aktiven Enzyms GlucDH nach 5 Wochen bei 32 °C und 85 % relative Luftfeuchtigkeit (Figur 7A). Das mit NAD stabilisierte Enzym weist nur noch eine extrem geringe Enzymaktivität auf (0,5 %), während das mit cNAD stabilisierte Enzym noch eine Restaktivität von 70 % besitzt (jeweils im Vergleich zu den mit Trockenmittel (TM) im Kühlschrank (KS) eingelagerten Proben). Nach 24 Wochen bei 32 °C und 85 % relativer Luftfeuchtigkeit (Figur 7B) beträgt die Restaktivität des Enzyms bei Stabilisierung mit cNAD noch immer etwa 25 %.

Auch die gelelektrophoretische Analyse von Glucose-Dehydrogenase im SDS-Gel (Figuren 8 und 9) zeigt deutlich den Unterschied zwischen einer Lagerung in Anwesenheit von NAD bzw. cNAD. Während das Enzym nach 5 Wochen Lagerung bei 32 °C und 85 % relativer Luftfeuchtigkeit in Gegenwart von cNAD noch als Bande mit der erwarteten Mobilität zu erkennen ist, ist das in Gegenwart von NAD gelagerte Enzym vollkommen verschwunden (Figur 8). Gleichzeitig ist aus Figur 9 ersichtlich, dass die Bande des mittels NAD stabilisierten und bei 50 °C gelagerten Enzyms mit zunehmender Lagerungsdauer schwächer wird und nach 476 Stunden nahezu verschwunden ist, während die entsprechende Bande des in Gegenwart von cNAD gelagerten Enzyms gegenüber einer zu Beginn des Experiments detektierten Bande eine lediglich geringfügige Änderung zeigt.

Dieses Ergebnis läßt sich auch bei Lagerung in flüssiger Phase bestätigen. (Figuren 10A und 10B): Nach 95 Stunden bei 50 °C liegt die Restaktivität von Glucose-Dehydrogenase in Gegenwart des nativen Coenzyms NAD bei = 5 %, während die Restaktivität der GlucDH in Gegenwart des artifiziellen Coenzyms cNAD bei 75 % liegt (Figur 10A). Nach 336 Stunden Lagerung bei 50 °C beträgt die Restaktivität des mit NAD stabilisierten Enzyms nur mehr etwa 1 %; für das in Gegenwart von cNAD gelagerte Enzym wird eine Restaktivität von immer noch etwa 70 % beobachtet. Die entsprechenden SDS-Gele zeigen ebenfalls eine Veränderung der GlucDH-Bande in Gegenwart des nativen Coenzyms NAD: es zeigen sich neue Banden bei höheren Molmassen und eine Verschiebung der 30 kDa-Bande.

Insgesamt ist es ein höchst überraschendes Ergebnis, dass die Stabilisierung des Cofaktors gleichzeitig eine Stabilisierung des Enzyms bewirkt - und dies nicht allein durch den kooperativen Effekt des besseren Zusammenhaltes des Enzyms. Der Zerfall des Cofaktors NAD hat einen negativen Effekt auf die Stabilität des Enzyms GlucDH und beschleunigt sogar dessen Inaktivierung. Der Austausch von nativem NAD durch künstliche Analoga erlaubt eine Lagerung der GlucDH unter Stressbedingungen (z.B. erhöhte Temperatur) auch in Gegenwart eines Cofaktors.

Mit einem solchen System lassen sich Blutglucose-Teststreifen mit erheblich verbesserten Stabilitätseigenschaften generieren, bei denen eine Darreichungsform ohne Trockenmittel möglich ist.

### Beispiel 2

Einer Alkoholnachweislösung wurden cNAD oder NAD zugesetzt. Diese Mischungen wurden bei 35 °C eingelagert. Anschließend wurde in regelmäßigen Abständen die Stabilität des Enzyms überprüft und eine Bestimmung der Restaktivität des Enzyms vorgenommen.

Wiederum zeigt eine gelelektrophoretische Analyse im SDS-Gel (Figuren 11 und 12) den Unterschied zwischen einer Lagerung in Anwesenheit von NAD bzw. cNAD. So unterscheiden sich die Banden der mit cNAD stabilisierten Alkohol-Dehydrogenase nach Lagerung über 65 Stunden bei 6 °C bzw. 35 °C nur geringfügig, was für eine Stabilisierung des Enzyms durch das artifizielle Coenzym spricht. Demgegenüber ist die Bande des in Gegenwart von NAD bei 35 °C gelagerten Enzyms vollkommen verschwunden (Figur 11).

Ferner wird aus Figur 12 deutlich, dass die Bande des mit NAD stabilisierten und bei 35 °C gelagerten Enzyms mit zunehmender Lagerungsdauer schwächer wird und nach 5 Tagen nahezu vollständig verschwunden ist. Eine nach 6 Tagen Lagerung bei 35 °C detektierte Bande des mit cNAD stabilisierten Enzyms zeigt eine deutlich geringere Zersetzung des Enzyms und damit eine erhöhte Stabilität.

Dieses Ergebnis läßt sich auch bei Lagerung in flüssiger Phase bestätigen. (Figur 13). Nach 65 Stunden bei 35 °C liegt die Restaktivität von Alkohol-Dehydrogenase in Gegenwart des nativen Coenzyms NAD bei etwa 6 %, während die Restaktivität des Enzyms in Gegenwart des artifiziellen Coenzyms cNAD noch bei etwa 60 % liegt.

## Patentansprüche

1. Verfahren zur Stabilisierung eines Enzyms,
**dadurch gekennzeichnet,**
**dass** man das Enzym in Gegenwart eines stabilen Coenzyms lagert.

2. Verfahren nach Anspruch 1,
**dadurch gekennzeichnet,**
**dass** das Enzym aus Dehydrogenasen ausgewählt wird.

3. Verfahren nach Anspruch 2,
**dadurch gekennzeichnet,**
**dass** dass das Enzym eine Dehydrogenase ist, ausgewählt aus einer Glucose-Dehydrogenase (E.C.1.1.1.47), Lactat-Dehydrogenase (E.C.1.1.1.27, 1.1.1.28), Malat-Dehydrogenase (E.C.1.1.1.37), Glycerin-Dehydrogenase (E.C.1.1.1.6), Alkohol-Dehydrogenase (E.C.1.1.1.1), alpha-Hydroxybutyrat-Dehydrogenase, Sorbitol-Dehydrogenase oder einer Aminosäure-Dehydrogenase, wie etwa L-Aminosäure-Dehydrogenase (E.C.1.4.1.5).

4. Verfahren nach einem der Ansprüche 1-3,
**dadurch gekennzeichnet,**
**dass** als Enzym eine Glucose-Dehydrogenase verwendet wird.

5. Verfahren nach einem der Ansprüche 1-4,
**dadurch gekennzeichnet,**
**dass** das stabile Coenzym aus stabilen Nicotinamid-Adenin-Dinucleotid-(NAD/NADH)- und Nicotinamid-Adenin-Dinucleotidphosphat-(NADP/NADPH)-Verbindungen ausgewählt wird.

6. Verfahren nach Anspruch 5,
**dadurch gekennzeichnet, dass** das stabile Coenzym aus Verbindungen mit der allgemeinen Formel (I) oder (II) ausgewählt wird: 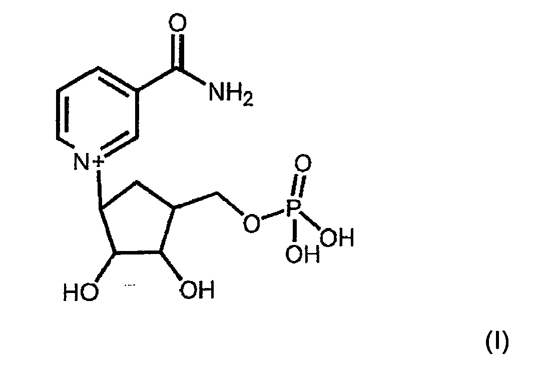 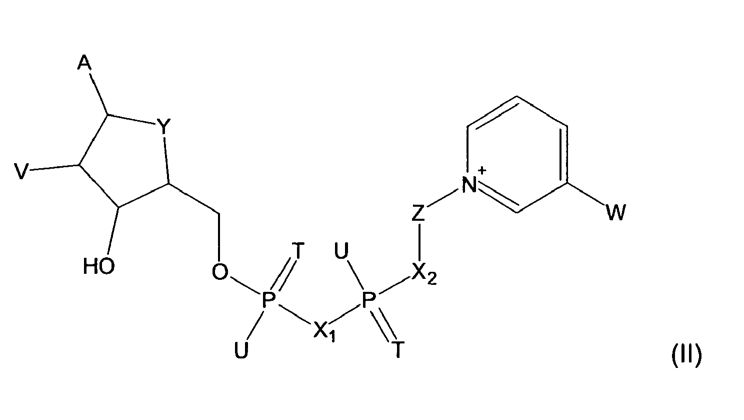 mit
A = Adenin oder ein Analog davon,
T = jeweils unabhängig O, S,
U = jeweils unabhängig OH, SH, BH₃-, BCNH₂⁻,
V = jeweils unabhängig OH oder eine Phosphatgruppe, oder zwei Gruppen, die eine cyclische Phosphatgruppe bilden;
W = COOR, CON(R)₂, COR, CSN(R)₂ mit R = jeweils unabhängig H oder C₁-C₂-Alkyl,
X¹, X² = jeweils unabhängig O, CH₂, CHCH₃, C(CH₃)₂, NH, NCH₃,
Y = NH, S, O, CH₂,
Z = ein linearer oder cyclischer organischer Rest ist, mit der Maßgabe, dass Z und der Pyridin-Rest nicht durch eine glycosidische Verbindung verknüpft sind, oder ein Salz oder gegebenenfalls eine reduzierte Form davon.

7. Verfahren nach Anspruch 6, worin Z ausgewählt wird aus
(i) einem linearen Rest mit 4-6 C-Atomen, vorzugsweise 4 C-Atomen, worin 1 oder 2 Atome gegebenenfalls durch ein oder mehrere Heteroatome ausgewählt aus O, S und N ersetzt sind, und
(ii) einem Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

8. Verfahren nach Anspruch 7, worin Z ein gesättiger oder ungesättigter carbocyclischer oder heterocyclischer Fünfring ist, insbesondere eine Verbindung der allgemeinen Formel (III) 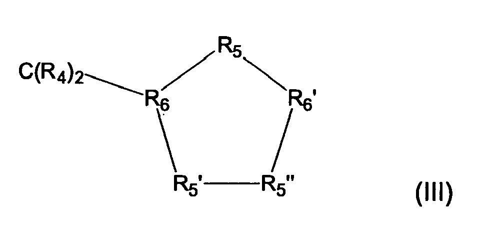
wobei zwischen R⁵' und R⁵" eine Einfach- oder Doppelbindung
vorliegen kann, mit
R⁴ = jeweils unabhängig H, F, Cl, CH₃,
R⁵ = CR⁴₂,
wobei R⁵' = O, S, NH, NC₁-C₂-Alkyl, CR⁴₂, CHOH, CHOCH₃, und
R⁵" = CR⁴₂, CHOH, CHOCH₃, wenn zwischen R⁵' und R⁵" eine Einfachbindung vorliegt,
wobei R⁵'=R⁵"=CR⁴, wenn zwischen R⁵' und R⁵" eine Doppelbindung vorliegt, und
R⁶, R⁶' = jeweils unabhängig CH oder CCH₃.

9. Verfahren nach einem der Ansprüche 6-8, worin W = CONH₂ oder COCH₃.

10. Verfahren nach Anspruch 8 oder 9, worin R⁵ CH₂ ist.

11. Verfahren nach einem der Ansprüche 8-10, worin R⁵' ausgewählt ist aus CH₂, CHOH und NH.

12. Verfahren nach einem der Ansprüche 8-11, worin R⁵' und R⁵" jeweils CHOH sind.

13. Verfahren nach einem der Ansprüche 8-11, worin R⁵' NH und R⁵" CH₂ ist.

14. Verfahren nach einem der Ansprüche 1-13,
**dadurch gekennzeichnet,**
**dass** man das mit einem stabilen Coenzym stabilisierte Enzym für eine Dauer von mindestens 2 Wochen, vorzugsweise von mindestens 4 Wochen, und besonders bevorzugt von mindestens 8 Wochen lagert.

15. Verfahren nach einem der Ansprüche 1-14,
**dadurch gekennzeichnet,**
**dass** man das mit einem stabilen Coenzym stabilisierte Enzym bei einer Temperatur von mindestens 20 °C, vorzugsweise von mindestens 25 °C und besonders bevorzugt von mindestens 30 °C lagert.

16. Verfahren nach einem der Ansprüche 1-15,
**dadurch gekennzeichnet,**
**dass** man das mit einem stabilen Coenzym stabilisierte Enzym ohne Trocknungsreagenz lagert.

17. Verfahren nach einem der Ansprüche 1-16,
**dadurch gekennzeichnet,**
**dass** man das mit einem stabilen Coenzym stabilisierte Enzym bei einer relativen Luftfeuchtigkeit von mindestens 50 % lagert.

18. Verfahren nach einem der Ansprüche 1-17,
**dadurch gekennzeichnet,**
**dass** die Lagerung des mit einem stabilen Coenzym stabilisierten Enzyms als Trockensubstanz erfolgt.

19. Verfahren nach einem der Ansprüche 1-17,
**dadurch gekennzeichnet,**
**dass** die Lagerung des mit einem stabilen Coenzym stabilisierten Enzyms in flüssiger Phase erfolgt.

20. Verfahren nach einem der Ansprüche 1-19,
**dadurch gekennzeichnet,**
**dass** die Lagerung des mit einem stabilen Coenzym stabilisierten Enzyms auf einem Testelement erfolgt.

21. Enzym, welches mit einem stabilen Coenzym stabilisiert ist,
**dadurch gekennzeichnet,**
**dass** bei einer Lagerung von vorzugsweise mindestens 2 Wochen, besonders bevorzugt mindestens 4 Wochen, und am meisten bevorzugt mindestens 8 Wochen bei einer Temperatur von vorzugsweise mindestens 20 °C, besonders bevorzugt mindestens 25 °C und am meisten bevorzugt mindestens 30°C, gegebenenfalls bei hoher Luftfeuchtigkeit und ohne Trockenreagenz eine Abnahme der enzymatischen Aktivität von weniger als 50 %, vorzugsweise weniger als 30 % und am meisten bevorzugt weniger als 20 % gegenüber dem Ausgangswert aufweist.

22. Nachweisreagenz zur Bestimmung eines Analyten, welches ein stabilisiertes Enzym nach Anspruch 21 enthält.

23. Testelement,
**dadurch gekennzeichnet,**
**dass** es ein stabilisiertes Enzym nach Anspruch 21 oder ein Nachweisreagenz nach Anspruch 22 enthält.
